# EUROPEAN PATENT APPLICATION

(11) **EP 0 792 661 A2**
(43) Date of publication of application: **03.09.1997**
(21) Application number: 95118293.0
(22) Date of filing: 21.11.1995
(51) Int. Cl.: A61M 39/00

(54) **Fluid connector**

(30) Priority: 11.10.1995 IL 11557395
(71) Applicant: PROMEDICAL S.r.l., 24056 Fontanella (Bergamo) (IT)
(72) Inventor: Barak, Swi, Ceasaria 38900 (IL)
(74) Representative: Gervasi, Gemma, Dr.

(57) **Abstract**

The invention refers to a modified Luer or Luer lock connector which can be used many times to provide a sterile communication for medical purposes, for example for performing blood transfusions.

The male part (1) of said connector includes a protruding member (4) which is closed by a septum (5) made of rubber (or another elastomeric material); the female part (10) of said connector includes a cavity (12) wherein an eccentric hollow needle (7), enclosed within a membrane (8) made of rubber (or another elastomeric material), is provided.

## Description

### TECHNICAL FIELD

The present invention relates to a closed sterile connecting assembly or connecting means (in the following referred to as "a connector") and more particularly to a multi-use of such connector which is to be used to provide sterile communication for fluids, i.e. between tubing and containers of sterile contents.

The connector according to the invention refers only to Luer and Luer lock connectors.

Though the main use of the connector would be for medical purposes, the same connector could be used also for any other fluids as, for example, corrosive fluids or fluids which are (or could be) noxious or in any way dangerous for persons and/or for the environment.

### BACKGROUND OF THE INVENTION

Connectors of the above kind are well known and in use in hospitals to perform blood transfusions or for the delivery of a sterile solution to a patient.

They comprise a male and a female part: within one of said parts is placed a piercing needle enclosed within an elastomeric membrane or sheath.

US-A-5,122,123 (Vaillancourt) refers to a connector formed with a male and a female part; the male part lumen is closed by a rubber septum, while the female part comprises a hollow needle mounted on the center of the female part and surrounded by a collapsible tube made of an elastomeric material and integral with a second rubber septum.

When the male part is inserted into the female one, its septum presses the septum of the female part against the needle, which pierces both septa; at the same time the collapsible tube surrounding the needle is compressed against the internal wall of the female part.

When the two parts are disconnected, the collapsible tube should expand to its unrestrained position covering again the needle and displacing the second septum in spaced relation to the needle, but the collapsed tube could be "stopped" by friction against the internal wall of the female part: it is therefore not sure that, when the two parts are disconnected, the collapsible tube expands always to its unrestrained position, covering in due time the needle to avoid any risk for a pratictioner to be accidentally pierced by the still exposed needle.

US-A-4,201,208 (Cambio, Jr.) refers to a connector which provides a sterile connection between a container for sterile solutions and a tubing for delivery of a solution; the container is provided with closure means including piercable diaphragms and the tubing is provided with a hollow pin surrounded by a sheath comprising a shoulder.

When the tubing is connected to the container, the external surface of the closure means engages itself with the sheath shoulder, pressing the sheath against the pointed end of the pin which therefore pierces the sheath.

US-A-4,673,400 (Martin) refers to an aseptic connector for conduits for sterile fluids comprising male and female parts provided with sealing membranes at their ends which must however be dipped in an antiseptic solution prior to their mating.

The male and female parts of Martin's connector are connected to each other simply by inserting the one into the other.

Finally, a Luer lock connector comprises a male and a female part connected to each other by screwing: the male part includes an inner member protruding therefrom and the female part includes an inner cavity wherein a hollow needle (to be inserted into said inner member of the male part during the connection procedure) is positioned: the two parts of a known Luer lock connector are "normally open" which means that, when disconnected, they remain exposed and are thus contaminated and the connector cannot therefore be reused.

Object of the present invention is a modified Leur lock connector of a "normally closed" type, which remains sterile even when its parts are disconnected, as will become apparent from the following description.

### DISCLOSURE OF THE INVENTION

The present invention refers to modified Luer lock connector where the protruding member of the male part is closed by a septum made of rubber (or another elastomeric material) and the hollow needle provided in the inner cavity of the female part is enclosed within a membrane made of rubber (or another elastomeric material) and is off-center mounted.

### BRIEF DESCRIPTION OF DRAWINGS

The invention will be better described with reference to the embodiment illustrated in the enclosed drawings where:
- figure 1 is a sectional view of the male part of the connector;
- figure 2 is a partially sectioned view of the female part of the connector;
- figure 3 is a partially sectioned view of the connector, assembled by connecting to each other its male and female parts.

### DETAILED DESCRIPTION OF INVENTION

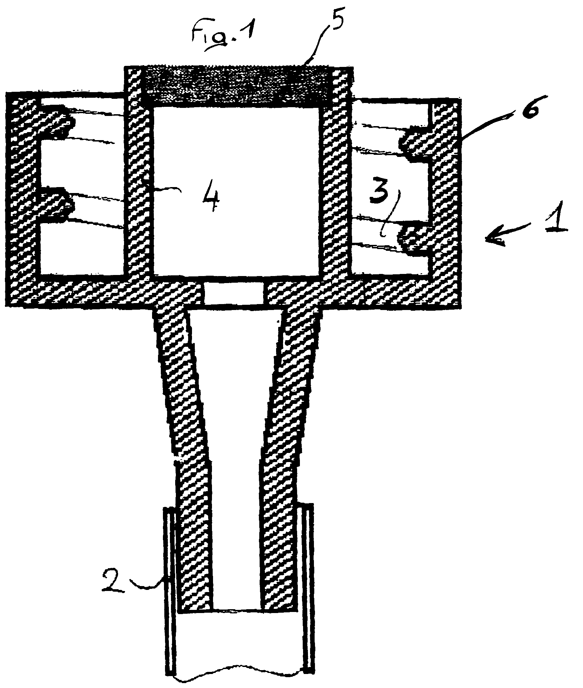

As shown by the sectional view of figure 1, the male part 1 of the modified Luer lock connector comprises a body 6 to one end of which in a normal manner a tube 2 is connected; the other end of the body 6 (to be connected by screwing to the female part 10 of the connector) is provided with threads 3 and with an inner cylindrical protruding member 4 closed by a rubber septum 5.

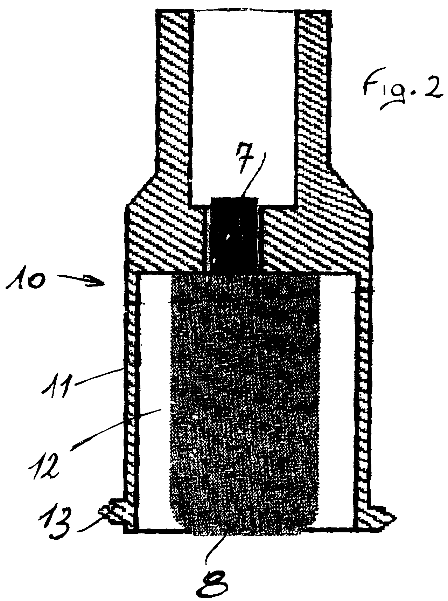

As shown by the partially sectioned view of figure 2, the female part 10 of the modified Luer lock connector comprises a body 11 provided with a screw thread 13 on its exterior surface and with an inner cavity 12 wherein an off-center mounted (eccentric) hollow needle 7, enclosed within a rubber membrane 8, is mounted; as in figure 2 no sectional view of the membrane 8 is provided, only the portion of the eccentric needle 7 "drowned" into the body 11 of the female part 10 is shown, while the remaining portion of the eccentric needle 7, including its pointed end, is covered by the membrane 8.

Of course, it is obvious for a skilled man to use (instead of rubber) any elastomeric material equivalent to rubber when realising the septum 5 and/or the membrane 8.

The eccentric needle 7 must be inserted into the inner member 4 of the male part 1 during the connection procedure of the male and female parts and the eccentricity of the needle 7 could not therefore exceed one half of the inner diameter of the inner member 4: taking account of the diameter of the needle 7, its eccentricity must be smaller then one half of the inner diameter of the inner member 4.

On a preferred embodiment of a Leur lock connector according to the invention, where the inner diameter of the inner member 4 of the male part 1 is of about 3 mm, the eccentricity of the needle 7 can vary from 0,2 and 0,5 mm.

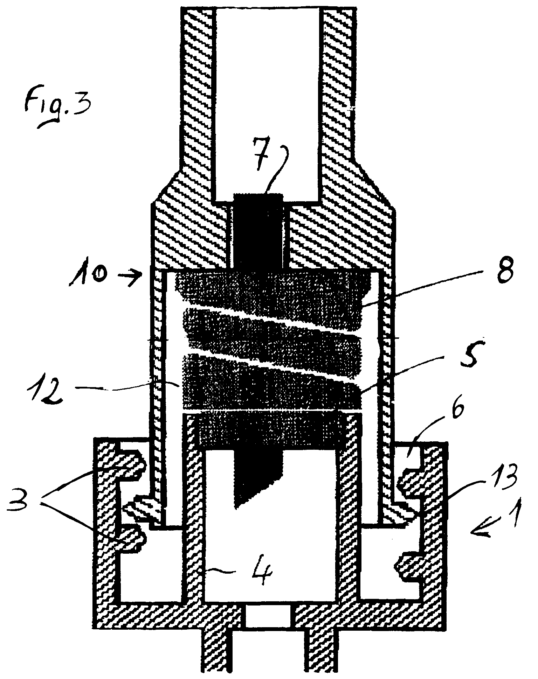

Figure 3 shows a partially sectioned view of the connector: to assemble its male and female parts (1, 10) to each other, the female part (10) is screwed into the male part (1) by engaging its screw thread 13 with the threads 3 provided inside the body 6 of the male part 1.

Consequently, as shown in figure 3 the eccentric needle 7 pierces through the septum 5 closing the inner member 4 of the male part 1 and the membrane 8 surrounding the eccentric needle 7, establishing a communication between the volumes connected to said two parts (1, 10), for example through the tube 2 shown in figure 1 and through another tube, not shown in figure 2, connected in a normal manner to the end of the female part 10 not engaged with the male one 1.

As the needle 7 is off-center mounted on the cavity 12 of the female part 10, it pierces the septum 5 and the membrane 8 in points which are always different in a random manner: the piercing of the septum 5 and of the membrane 8 in different random points (instead of always in the same points, as occurs in known devices) allows a longer multi-use of the connector and avoids any risk of affecting its sterility.

As shown by figures 1 and 2, the inner member 4 of the male part 1, closed by the septum 5, is protruding from the body 6 of the male part 1, and the upper surface of the membrane 8 is almost at the same level as the upper surface of the body 11 of the female part 10.

During the assembling procedure, the male part 1 of the connector is first of all inserted into the female one 10 (pressing therefore the septum 5 against the membrane 8) and before the male and female parts (1, 10) are connected to each other by screwing: during the insertion step, a friction contact is established between the septum 5 and the membrane 8, which is engaged at the same time by the pointed end of the eccentric needle 7.

During the following screwing step, the septum 5 compels by friction the membrane 8 to be twisted by turning round the eccentric needle 7 (which serves therefore also as a turning axis) and to collapse to the center of the cavity 12 of the female part 10 as clearly shown in figure 3, preventing any friction on the internal wall of the cavity 12 of the female part 10 and avoiding therefore any risk that, when the two connector parts (1, 10) are disconnected, the coming back of the membrane 8 to its initial position could be "slowed" or "stopped" by friction against the internal wall of the cavity 12 of the female part 10.

In addition, during the assembling procedure of the male and female parts (1, 10) of the connector, the septum 5 "loads" like a spring the elastomeric membrane 8 through a first elastic force P1 (due to the longitudinal displacement of the two parts) and through a second elastic force P2 due to the twisting of the elastomeric membrane 8 turning round the eccentric needle 7.

When disconnecting the male and female parts (1, 10), the total force (P1 + P2) is released by the "spring loaded" twisted elastomeric membrane 8, which is forced by said total force to return to its initial position; as P2 is always stronger than P1 (the twisting stress is higher than that due to the longitudinal displacement), the membrane 8 remains in close contact with the septum 5 even at the end of the unscrewing step: the needle 7 is therefore inside the membrane 8 when the septum 5 and the membrane 8 are still in close contact.

It is therefore sure that, when the two parts (1, 10) of the connector are disconnected, the membrane 8 always expands quickly to its unrestrained position, covering the needle 7 and avoiding any risk that a pratictioner could be accidentally pierced by a still exposed needle 7; in addition, no drops of the liquid (for example blood or another potentially dangerous liquid) which has flowed through the connector could infiltrate between the external surfaces of the septum 5 and of the membrane 8, which are still in close contact: any risk for a pratictioner to be contaminated by said liquid is therefore avoided and the safety of the pratictioner handling a used connector is therefore further increased.

The above are relevant features of the connector according to the invention, which overcomes therefore the above mentioned disadvantages of Vaillancourt's devices and of the other known connectors.

During the screwing step, the eccentric needle 7 (which is integral to the female part 10) has a rotational movement (which co-operates with the friction contact between the septum 5 and the membrane 8 to twist the membrane 8) and a longitudinal displacement: to avoid any risk that the eccentric needle 7 could damage the septum 5 during its rotational movement, the needle 7 must pierce the septum 5 only at the last stage of the screwing step.

To establish communication between the two volumes connected to the male and female parts (1, 10) when the connector is assembled, it is enough that (as shown in figure 3) only the pointed end of the eccentric needle 7 protrudes into the inner member 4 of the male part 1: to this purpose, on a preferred embodiment of a Leur lock connector according to the invention the pointed end of the eccentric needle 7 is located from 3 to 4 mm below the upper surface of the body 11 of the female part 10 (for example, if the cavity 12 of the female part 10 is about 12 mm length the eccentric needle 7 is about 8 mm length), the reciprocal longitudinal displacement of the male and female parts (1, 10) during the screwing step is of about 4 mm, the inner member 4 protrudes from the body 6 of the male part 1 from 1,5 to 2 mm, the septum 5 is about 2 mm thick and the membrane 8 is about 3 mm thick.

## Claims

1. A connector of the Luer lock type which comprises male and female parts (1, 10) connected to each other by means of a thread arrangement (3, 13), where the protruding member (4) of said male part (1) is closed by an elastomeric septum (5) and where in the inner cavity (12) of said female part (10) a hollow needle (7) enclosed within an elastomeric membrane (8) is provided, characterised in that said needle (7) is off-center mounted.

2. A connector as claimed in claim 1 where said male and female parts (1, 10) are connected to each other by inserting said male part (1) into said female part (10) and before by screwing to each other said male and female parts (1, 10) by means of said thread arrangement (3, 13), characterised in that, during said insertion step, said elastomeric septum (5) is pressed against said elastomeric membrane (8) and a friction contact between said elastomeric septum (5) and said elastomeric membrane (8) is therefore established.

3. A connector as claimed in claim 2, characterised in that said elastomeric septum (5) compels through said friction contact said elastomeric membrane (8) to be twisted by turning around said off-center mounted needle (7) during said screwing step.

4. A connector as claimed in claim 3, characterised in that said elastomeric membrane (8) collapses to the center of said inner cavity (12) of said female part (10) when twisting.

5. A connector as claimed in claim 1, characterised in that the eccentricity of said off-center mounted needle (7) is smaller then one half of the inner diameter of said protruding member (4) of said male part (1).

6. A connector as claimed in claim 5, characterised in that the eccentricity of said off-center mounted needle (7) is comprised between 0,2 and 0,5 mm.

7. A connector as claimed in claim 1, characterised in that, when said male and female parts (1, 10) are connected to each other, said off-center mounted needle (7) pierces in different random points said elastomeric septum (5) closing said protruding member (4) of said male part (1) and said elastomeric membrane (8) surrounding said off-center mounted needle (7).

8. A connector as claimed in claim 1, characterised in that said off-center mounted needle (7) pierces said elastomeric septum (5) closing said protruding member (4) of said male part (1) only at the last stage of said screwing step.

9. A connector as claimed in claim 1, characterised in that only the pointed end of said off-center mounted needle (7) protrudes into said inner member (4) of said male part (1) when said connector is assembled.

10. A connector as claimed in claim 2, characterised in that the pointed end of said off-center mounted needle (7) is located from 3 to 4 mm below the upper surface of said female part (10), the reciprocal longitudinal displacement of said male and female parts (1, 10) during said screwing step is of about 4 mm, said protruding member (4) protrudes from 1,5 to 2 mm from said male part (1), said elastomeric septum (5) closing said protruding member (4) of said male part (1) is about 2 mm thick and said elastomeric membrane (8) surrounding said off-center mounted needle (7) is about 3 mm thick.
